# EUROPEAN PATENT APPLICATION

(11) **EP 0 965 278 A1**
(43) Date of publication of application: **22.12.1999**
(21) Application number: 98830365.7
(22) Date of filing: 16.06.1998
(51) Int. Cl.: A23L 1/308

(54) **Dietetic food composition and dietetic method using such composition**

(71) Applicant: Zohoungbogbo, Mathias Christian, 10040 Rivalta Di Torino (IT); Gobbato, Rosa Anna, 10040 Rivalta di Torino (IT)
(72) Inventor: Zohoungbogbo, Mathias Christian, 10040 Rivalta Di Torino (Torino) (IT)
(74) Representative: Ferreccio, Rinaldo

(57) **Abstract**

Food composition in the form of a flour comprising at least 50% of protein, less than 15% of carbohydrates and 35 to 50% of plant fibres; preferably the carbohydrate content is less than 10%, advantageously less than 5%; this composition may be used as a substitute for wheat flour in the preparation of foods such as pasta, bread, bread sticks, bakery products and pastries and constitutes the basis of a method for improving the appearance of a person by achieving a loss of weight which is beneficial from the aesthetic point of view.

## Description

This invention relates to the sector of the food and dietetic industry.

In particular, the invention relates to a substitute food composition for cereal flours in general and for wheat flour in particular, and foods which can be prepared using this composition.

The invention also relates to a method for controlling the body weight of an individual.

It is well known that in the developed nations and even in the developing nations the percentage of individuals having excess weight if not obesity problems is constantly and progressively increasing. This has important consequences both for the health of individuals and for the overall health cost of the various nations, because it has been amply demonstrated that obesity or even being overweight are important associated causes of cardiovascular and metabolic diseases, such as myocardial infarction, stroke, type II diabetes, etc.

The dietetic measures generally proposed by dieticians to combat and/or prevent excess weight mainly consist of low-calorie or low-fat regimes.

Another widely recommended measure for the control of body weight which is even advocated by the mass media (periodicals, television, etc.) is adoption of the famous "Mediterranean diet", based on foods rich in complex carbohydrates such as pasta, rice, bread and the like.

It is, however, a fact that the widespread use of low-calorie diets and the Mediterranean diet has not in fact resulted in any statistically significant change in the percentage of obese or overweight persons; there is instead a progressive increase in this percentage.

A low-calorie diet can in fact have some temporary effect on reducing body weight but this cannot be maintained for a long period, either because it results in general weakening of the body, or because over the long term it is rejected by the individual because of the monotony of the flavours of the food making it up (essentially meat, fish and greens).

The so-called "Mediterranean diet" is in fact only suitable for maintaining the right weight and the right form in individuals who are engaged in vigorous physical activity. Persons who are engaged in essentially sedentary work can on the other hand experience an increase in weight and an accumulation of lipids when they feed themselves on foods based essentially on carbohydrates.

The problem underlying this invention is that of providing a diet/foodstuff which allows obese or merely overweight persons to recover their ideal weight by eliminating excess lipids and to maintain such an ideal weight over a long period of time. All this without the persons having to subject themselves to a low-calorie diet and to suffer the deprivation of foodstuffs which it is difficult to do without over long periods, such as pasta, bread and bakery products in general.

Such a problem is solved according to the invention by a food composition in the form of a flour comprising at least 50% of protein, less than 15% of carbohydrates and from 35 to 50% of plant fibres.

Preferably the carbohydrate content is less than 10%, advantageously less than 5%.

The proteins are preferably selected from the group comprising gluten, soya proteins, milk proteins, animal proteins obtained from meat or dried or smoked fish, egg albumen, wheat germ, rice germ.

The plant fibres are preferably selected from the group comprising cereal fibres, in particular wheat, maize and oats, vegetable fibres, in particular tomato and spinach, and fruit fibres, in particular oranges and apples.

Both the proteins and the plant fibres are used in a finely divided form and mixed in suitable ratios to produce flours which can be used as substitutes for wheat flour in the preparation of foods such as pasta, bread, bread sticks, bakery products and pastries.

This invention also relates to a method for improving the appearance of a person by achieving a loss of weight which is beneficial from the aesthetic point of view, this method comprising the elimination from the said person's diet of all carbohydrate-based foods and their replacement with foods obtained using the flours described above.

Advantageously such a method provides for the initial use of flours having the lowest carbohydrate content possible, in any event a content of not more than 5% by weight.

A certain although minimum carbohydrate content is always present in the flours according to the invention because commercially available plant fibres always have a small residual glucide content. Once the desired aesthetic effects have been achieved, it is then possible, according to the method of the invention, to use flours with a higher carbohydrate content in a second stage. Preferably flours with a carbohydrate content of less than 15% should be used.

In a third stage of the method, which can be described as a maintenance stage, the carbohydrate content of the flours can be further increased, without however exceeding the threshold of 20% by weight. This guarantees maintenance of the aesthetic effects obtained, while at the same time introducing greater variety into the diet.

The method according to the invention therefore provides for an individual who intends to lose weight to eliminate all carbohydrate-rich foods such as bread, pasta, sweets and bakery products in general from his diet and to replace them by "facsimile" foods obtained using the flours described above, using non-sugar sweeteners in the case of sweets.

The abovementioned flours may be used in the same way as wheat flour. To prepare bread for example, such flours are made into a dough with water and yeast, and possibly salt, lard, olive oil or other optional ingredients, allowed to rise and then baked in an oven at the same temperature at which bread is baked.

Similarly, in order to prepare sweets such as tarts, biscuits or the like, the flours are made into a dough with butter or margarine or similar fats, eggs, yeasts if desired and other optional ingredients, with the addition of non-glucide sweeteners (e.g. saccharine) and baked in an oven in the same way as traditional sweets.

Those who intend to regain their ideal weight by following the method according to the invention should also abstain from consuming fruits which are rich in sugars, sweetened drinks, etc.

The food composition and the method according to this invention will be further described with reference to the examples provided below merely by way of non-restrictive illustration in which the percentages stated are to be understood as percentages by weight of the total dry weight.

### EXAMPLE 1

| | |
|---|---|
| Wheat gluten | 55% |
| Wheat fibre | 40% |
| Vegetable fibres | 5% |

By wheat fibre is meant the fibre of the wheat stems (one example of a commercially available product is that sold by the name of VITACEL®).

Wheat gluten is a product marketed by various firms such as for example the company Rocchetta (Italy).

The vegetable fibres are for example fibres of tomato, spinach, onion, etc.; these fibres are marketed by various firms, including Newfood (Italia).

### EXAMPLE 2

| | |
|---|---|
| Wheat gluten | 35% |
| Soya proteins | 20% |
| Wheat fibre | 45% |

An example of a commercially available product comprising soya protein is Soymin®.

### EXAMPLE 3

| | |
|---|---|
| Wheat gluten | 35% |
| Fruit fibres | 45% |
| Soya proteins | 20% |

The fruit fibres used in this composition were those marketed in Italy under the name VITACEL Arancia® and VITACEL Mela®.

### EXAMPLE 4

| | |
|---|---|
| Wheat gluten | 34% |
| Wheat germ | 33% |
| Wheat fibre | 17% |
| Vegetable fibres | 16% |

Wheat germ is marketed by various producers, such as for example the company Rizzolio (Italy).

### EXAMPLE 5

| | |
|---|---|
| Milk proteins | 10% |
| Wheat fibre | 15% |
| Wheat germ | 60% |
| Fruit fibres | 15% |

### EXAMPLE 6

The flour in Example 1 was used to prepare pasta. For this purpose the flour was mixed with a sufficient quantity of water to obtain a dough suitable for forming by extrusion (approximately 20 to 30% of water). A pasta in the form of spaghetti was obtained by extrusion through suitable dies and when subsequently cooked in boiling salted water yielded organoleptic properties entirely similar to those of a pasta made with high quality durum wheat semolina.

### EXAMPLE 7

The flour in Example 2 was used to prepare bread. For this purpose the flour was mixed with a sufficient quantity of water to obtain a mouldable dough, and to this a suitable quantity of bread yeast and salt were also added. The dough was then left to rise for approximately 2 hours, mixed again and finally left to stand for approximately 1 hour before being shaped and baked in an oven at approximately 220°C.

The bread obtained in this way had optimum organoleptic properties, crustiness and crumbliness.

The flours in the examples described above are virtually interchangeable and can be used in the preparation of pasta, egg pasta, bread, bread sticks, biscottes, sweet and savoury flans, biscuits, etc., following the addition of suitable additional ingredients.

The only differences which can be found between the various flours lie in their different carbohydrate contents, which for example are lowest in the flour according to Example 1 (definitely below 5%) and tend to be higher (although always below 15%) in the flours containing fruit or vegetable fibres, on account of the residual glucide content of those fibres.

As a consequence a person who wishes to lose weight by following the method according to this invention should initially use flours such as those in Examples 1 or 2, which guarantee an absolutely negligible glucide content.

Once the ideal weight has been gained the individual can alternate the use of the abovementioned flours with those of flours (such as those in Examples 3 and 4) which have a certain, though low, glucide content due to the fruit or vegetable fibre content.

Once the individual's weight and form have become stabilized, then flours containing other ingredients such as milk proteins, dried meat or fish animal proteins, powdered milk, powdered cream, whole eggs or dried albumin or dried egg yolk only can also be introduced into the diet.

In this latter stage the carbohydrate content should however always be maintained below 20% by weight.

It should be pointed out that the flours according to the invention constitute the basis of the food intake of an individual who intends to improve his appearance by eliminating excess kilos using the method according to this invention. Provided that all carbohydrate-based foods are replaced by similar foods prepared using the flours according to the invention the individual can nevertheless consume a variety of foods rich in proteins and/or lipids (meat, fish, delicatessen products, cheeses) without any limit on quantity and without in any way prejudicing the result achieved, or losing excessive weight.

This constitutes an enormous advantage in comparison with the low-calorie diet regimes proposed hitherto, which presuppose a considerable propensity to "sacrifice" on the part of individuals who subject themselves to them, and which for this reason have therefore as a result achieved very low compliance on the part of such individuals.

In addition to imposing no quantitative limits on food intake, the method according to the invention makes it possible to vary foods consumed with great freedom, which further increases the willingness of individuals to follow the proposed diet regime satisfactorily.

## Claims

1. A food composition in the form of a flour comprising, as percentages by weight of the total dry weight, at least 50% of protein, less than 20% of carbohydrates and 30 to 50% of plant fibres.

2. A food composition according to Claim 1, in which the carbohydrate content is less than 15%.

3. A food composition according to Claim 2, in which the carbohydrate content is less than 5%.

4. A food composition according to any one of the foregoing claims, in which the proteins are preferably selected from the group comprising gluten, soya proteins, milk proteins, animal proteins obtained from meat or dried or smoked fish, egg albumen, wheat germ, rice germ.

5. A food composition according to any one of the foregoing claims, in which the plant fibres are selected from the group comprising the fibres of cereals, in particular wheat, maize and oats, vegetable fibres, in particular tomatoes and spinach, and fruit fibres, in particular oranges and apples.

6. A substitute food for pasta obtained using the conventional techniques and the conventional equipment used for the production of pasta, substituting the flour or wheat semolina with a flour according to any one of Claims 1 to 5.

7. A substitute food for bread or similar bakery products obtained using the conventional techniques and the conventional equipment used for the production of bread and similar bakery products, replacing the wheat flour with a flour according to any one of Claims 1 to 5.

8. A method for improving the appearance of a person by achieving a loss of weight which is beneficial from the aesthetic point of view, this method comprising the elimination from the said person's diet of all carbohydrate-based foods and their replacement with foods obtained using the food compositions according to any one of Claims 1 to 5.

9. A method according to Claim 8, characterized in that it comprises:
an initial stage in which the said carbohydrate-based foods are replaced with foods obtained using food compositions according to Claim 3.
an intermediate stage in which the said carbohydrate-based foods are replaced with foods obtained using food compositions according to Claim 2, and
a maintenance stage in which the said carbohydrate-based foods are replaced with foods obtained using food compositions according to Claim 1.
